# EUROPEAN PATENT APPLICATION

(11) **EP 2 674 114 A1**
(43) Date of publication of application: **18.12.2013**
(21) Application number: 12171550.2
(22) Date of filing: 11.06.2012
(51) Int. Cl.: A61B 17/12, B29C 53/60

(54) **Implant for occlusion of vascular anomalies and method for producing such an implant**

(71) Applicant: Acandis GmbH & Co. KG, 76327 Pfinztal (DE)
(72) Inventor: Watson, David, San Jose, CA California 95125 (US)
(74) Representative: Kilchert, Jochen

(57) **Abstract**

Provided is an implant (10) for occlusion of vascular anomalies comprising a flexible element that is formed from a primary helical winding (11), wherein at least a portion of the primary helical winding is composed of at least two wires (12,13) forming at least two independent helical coils as a multiple helix winding, the wires (12,13) having different properties. Further, the wires (12,13) having different properties such as different material and further wherein one of the wires (12,13) is made from a radiopaque material and another one of the wires (12,13) is made from a non-radiopaque material providing a distinguished implant (10). The present invention also provides a method for producing an implant (10) for occlusion of vascular anomalies.

## Description

### Field of the Invention

The invention relates to the field of vaso-occlusive devices for the treatment of various vascular anomalies such as for example aneurysms, arterio-venous fistula, and artery occlusions. More particularly, the invention provides an implant that after deployment conforms to the shape of an anatomical cavity. Further, the invention relates to a method of producing such an implant.

### Background of the Invention

The treatment of vascular anomalies such as aneurysms, arterio-venous fistula, and artery occlusions or other vascular anomalies remains an important area of research both to physicians and patients alike and has improved dramatically in recent years by placement of devices within a vasculature of a patient such that many vascular anomalies are treated in-situ without an open surgical procedure.

In general, vaso-occlusion devices are therapeutic devices that are placed within the vasculature of the human body, typically via a catheter, either to block the flow of blood through a vessel making up that portion of the vasculature through the formation of an embolus or to form such an embolus within an aneurysm stemming from the vessel, thus ultimately healing the aneurysm.

Moreover, such devices allow a physician to treat a patient non-invasively so that exposure to risk factors associated with invasive surgery is minimized. That is, the placement of vaso-occlusive devices within a vasculature of a patient allows the physician to treat a patient without using surgical techniques that are considered a higher risk to the patient.

These vaso-occlusive devices can be produced in such a way that they will pass through the lumen of a catheter in a linear shape and take on a complex shape as originally formed after being deployed into the area of interest, such as an aneurysm. These devices are commonly produced as primary coiled winding of an implantable metal that is subsequently formed into a more complex secondary shape by winding the primary winding about poles placed on a winding mandrel. The secondary wound coil is then annealed on the winding mandrel, and the coil is then removed from the winding mandrel. One widely used vaso-occlusive device as described in US patent 5,645,558 is a helical wire coil having a deployed configuration which is generally spherical shaped and can be dimensioned to engage the walls of the vessels or aneurysm. The delivery of such vaso-occlusive devices can be accomplished by a variety of means, including via a catheter in which the device is pushed through the catheter by a pusher to deploy the device. The embolic coil is releasably attached to a pusher member such that the physician may manipulate the position of the coil prior to detaching it in-situ. Various detachment mechanisms to release the device from a pusher have been developed and are known in the art.

Another exemplary implantable vaso-occlusive device is disclosed in US 5,749,891. The device has multiple secondary layers formed from a continuous primary winding of a single wire wherein the final shape is a generally spherical or ovoid coil formed of linear or helical primary coil that is wound into a secondary form having three layers. The inner winding is wound and then the second layer formed by winding in the opposite direction of the first layer. However, such a device suffers from not being well suited to conform to a variety of aneurysm shapes.

The primary winding of these micro-coils is generally formed from a very small metallic wire with a diameter generally ranging from 40 to 80micrometers. This wire is formed into a primary coil generally about 0.25mm - 0.50mm in diameter such that it can be inserted in a linear configuration up a correspondingly sized microcatheter. A variety of materials have been suggested for use in such micro-coils, including nickel-titanium alloys, stainless steel, platinum, tungsten, various plastics or the like, each of which offers certain benefits in various applications. Platinum alloys are particularly advantageous for the fabrication of such micro coils, in that they can have suitable shape memory properties, radiopacity to be seen with x-ray during deployment, and can be manufactured to easily fit into a linear portion of a catheter, yet attain their originally formed, more complex shape when deployed. However, as Platinum alloys are a precious metal, the cost of such coils is generally quite high. Also, since numerous coils are often needed to treat a particular vessel, the high radio-density of the Platinum in the vessel creates an imaging artefact that is often undesirable. Therefore it has been desirable to find a means to fabricate these embolic devices with the necessary radiopacity while maintaining the desirable softness and shape memory qualities necessary. To achieve clinical efficacy while reducing the amount of Platinum present in the coil, or to reduce the number of coils needed for a particular treatment some methods have previously been described.

One described vaso-occlusive coil is known from US 6,638,291, for example, that has a three dimensional in-filling coil configuration, formed of a micro-cable comprised of a multiplicity of braided wires wound around a central wire. This primary micro-cable is then wound into a secondary similar in fashion to a conventional micro coil. These described vaso-occlusive devices are generally constructed from a primary micro-cable that is formed from multiple wires of various materials wound around a central core wire that remains part of the micro-cable. However, due to the natural stiffness of these composite cables, these implants would generally be too stiff and could lead to vessel rupture. Embolic device coils constructed in this manner have never been introduced to the market.

In publication United States Patent 7,608,089, an assembly for occluding a vascular site (e.g., an aneurysm) of a human or veterinary patient is disclosed which includes a vaso-occlusive member, a pusher member having a distal end and a severable junction located proximal to the distal end, and a pivotable coupling that couples the pusher member to the occlusive member. However, one of the coils of the double helix is only used for mounting purposes. Moreover, the two coils are fixedly attached to each other and are therefore not independent from each other.

Yet another conventional implant for vessel occlusion as disclosed in US Patent 7,842.054 is made from a primary helical metal element that has an outer layer of expansive hydrogel material applied to its outer surface. The purpose of the outer hydrogel is to expand in-situ thus providing for a greater filling volume within the aneurysm. In controlled clinical trials, these coils demonstrated a reduction in recurrence and retreatment compared to non-hydrogel coated coils, however there was an unexplained increase in the incidence in hydrocephalus with the use of these coils in patients that did not have subarachnoid haemorrhage from ruptured aneurysms. [Philip M. White, MD, et al in The Lancet (2011:377:1655-1662)]. Therefore it would be desirable to provide for greater filling density to reduce the number of costly coils used without the side-effects that may be related to a bio-reactive polymer.

Embolic coils formed from primary winds consisting of the generally used Platinum-Tungsten (Pt-W) alloy, are easily unravelled if a tensile load is applied to the coil, such as during repositioning within an aneurysm by the physician.

For this reason, many embolic coils have 'stretch resistant' fibers added within the central lumen of the primary wind to prevent such unravelling. The means to achieve this is well known in the art, and exemplified in US Patent 5,582,619. However, the cost to add these fibres due to the labour intensive nature of the process is significant. Hence, an expensive means to improve the stretch resistance of Platinum alloy embolic coils is yet to be found.

From the foregoing, it can be seen that vaso-occlusive devices have provided important improvements in occlusion devices for the treatment of aneurysms and various types of arteriovenous anomalies, but there remains important limitations in the technology presently available.

It is an object of the present invention to provide an implant that is improved as regards the handling by a physician when delivered to the site to be treated. In particular, the present invention provides an implant that efficiently reduces resource material by providing a greater filling volume within the aneurysm. Moreover, it is an object of the present invention to provide a method for producing such an implant.

### Summary of the Invention

This object is achieved by the implant according to claim 1. As regards the method said object is achieved by the subject matter of claim 13.

The present invention provides an implant for occlusion of vascular anomalies comprising a flexible element that is formed from a primary helical winding, wherein at least a portion of the primary helical winding is composed of at least two wires forming at least two independent helical coils as a multiple helix winding. The at least two wires may preferably have different properties and are preferably arranged in an alternating fashion along the length of the primary helical winding when viewed in a longitudinal cross section.

Each independent helical coil may have distal and proximal ends disposed along the length of the flexible element, wherein at least the proximal ends of each independent coil are constrained generally co-axially to form the flexible element. Furthermore the at least two independent coils may be constrained to have a common lumen by a constraining means, in particular a stretch resistant fibre which may be provided within at least a portion of the common lumen to prevent complete disassociation of the at least two independent coils. The constraining means may be removable to promote disassociation of the at least two independent coils.

The at least two wires may be releasably attached along at least a portion of the flexible element and the at least two independent helical coils may be disposed generally co-axially in a delivery configuration and may be expandable to a non co-axial configuration when deployed. Each independent helical coil may have distal and proximal ends disposed along the length of the flexible element, wherein at least the proximal ends of each independent coil may be constrained generally co-axially to form the flexible element. In particular the at least two independent coils may be constrained to have a common lumen wherein a constraining means, in particular a stretch resistant fibre, may be provided within at least a portion of the common lumen to prevent complete disassociation of the at least two independent coils. The constraining means may be removable to promote disassociation of the at least two independent coils.

According to another embodiment of the invention, the at least two wires may have the same or different properties. The two windings may be configured for example to pre-stressing, such that when delivered from a delivery means such as a catheter the at least two individual coils partially dissociate their co-linear relationship and expand to fill a greater volume within the aneurysm or vessel. Therefore, the total number of implants needed for a procedure could be reduced thus lowering the total costs of treatment. From the forgoing, it can be seen that this embodiment of the invention could be achieved with wires of similar or dissimilar properties as long as the individual coils made from the independent wires are configured to partially dissociate their co-linear arrangement upon delivery.

One advantage resulting from the invention is that the wires may have different properties allowing for coil engineers more freedom to selectively influence the characteristics of the implant. For example the properties of one wire are selected to improve a first function of the implant and the properties of another wire are selected to improve a second function of the implant. Moreover, the characteristics of the implant can be better improved by the different properties of the wires than in the prior art because in the prior art said characteristics are determined by the properties of the single wire, for example by the material, used to form the primary helical winding. The wires having different properties may lead to a combined total characteristic of the implant that is improved over the characteristic of a single wire implant. Further the costs of the implant can be reduced while maintaining the desired properties of the implant at least to a certain extent. For example, one wire may be made of a less expensive material while another wire, that is more expensive, provides the desired properties of the implant. Owing to the side-by-side arrangement of the wires, the improved characteristics are substantially uniformly present along the length or at least along a partial length of the implant.

Preferred embodiments of the invention are indicated in the dependent claims.

The different properties of the wires may include the material of the wires. One of the wires may be made from a radiopaque material and another one of the wires may be made from a non-radiopaque material. This leads to a reduced radiopacity so that the x-ray artefact may be reduced thereby allowing better visibility of additional coils subsequently placed within the central body of others previously placed within an aneurysm.

By using different materials for the wires, the stretch resistance may be inherently enhanced. By use of two wires of dissimilar materials, one to provide for the required radiopacity (Pt-W) and another to provided for enhanced tensile yield properties, for example 316L stainless steel or Cobalt-Chromium, the natural tendency to unravel in tension is reduced, thereby possibly eliminating the need to add stretch resistant fibers that are labour-intensive and costly.

The different properties of the wires may however include the diameter of the wires. If only one of the two wires provide an external contact surface, the friction of the implant is reduced when delivered up a catheter to the site of a vascular anomaly.

The implant further may comprise helical coils wherein the adjacent coil loops are disposed in direct contact with each other (so as to achieve various performance objectives) or they may be disposed slightly set apart from each other (to achieve other performance objectives). Fabrication of the implant can be facilitated with either result. The flexible element may either be expandable to form a secondary configuration or may form a random configuration conforming to the shape of a vascular site when delivered to the vascular site. The concept of a primary helical winding at least partially composed of at least two wires is therefore applicable to manufacturing various novel implants with enhanced features dependent of the configuration of the wire materials and coil geometry configurations.

The present invention also provides a method for producing an implant for occlusion of vascular anomalies comprising forming a flexible element wherein at least a longitudinal portion of said element is composed of at least two independent helical coils formed into a multiple helix winding. The wires may be formed into coils by winding them onto the mandrel simultaneously or sequentially. The mandrel may be of uniform cross-section such that the wound wires of different properties have an outer profile which is different to an inner profile. The individual coils of the multiple helix winding may also be made sequentially on a series of mandrels by exchanging mandrels in subsequent winds such that the wound wires may have different properties, inner profiles or cross-sectional shapes.

### Brief description of the Drawings

A better understanding of the features and advantages of the present invention will be obtained by reference to the following detailed description that sets forth illustrative embodiments by way of example only, in which the principles of the invention are utilized, and the accompanying drawings of which:
Figure 1 shows a perspective view of an embodiment of a length-wise segment of an implant depicting a primary helical winding according to the present invention and composed of first and second wires disposed side-by-side forming helical coils as a multiple helix winding.
Figure 2 shows an end-on elevation view of the primary winding of Figure 1 according to an embodiment of the present invention.
Figure 3 shows a cut-away (along line A-A of figure 2) longitudinal section view of an embodiment of an implant according to the present invention showing first and second wires disposed side-by-side forming a helical winding.
Figure 4 shows a perspective view of one embodiment of a proximal end of the implant according to the present invention in which the proximal ends of the first wire and second wire of the helical winding are provided as attachment means.
Figure 5 shows a perspective view of one embodiment of a distal end of the implant according to the present invention in which the distal end of the first wire and second wire of the helical coils are encapsulated in a hemispherical solder mass to produce a non-traumatic distal tip.
Figure 6 shows a cross-section of the implant after implantation in which the two coils have dissociated their co-linear relationship and are lengthwise constrained from further dissociation by a constraining means arranged within a common lumen according to an embodiment of the present invention.
Figure 7 shows an end-on elevation view (along line B-B of Figure 6) in which the dissociated co-linear relationship of the two coils are further shown with a constraining means arranged within a common lumen according to an embodiment of the present invention.

### Detailed Description of the Invention

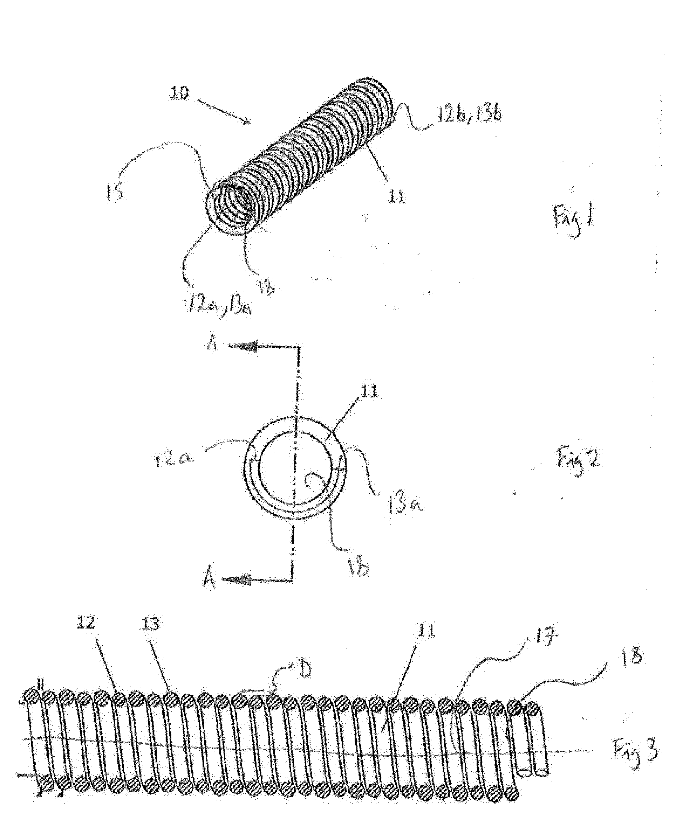

Figure 1 shows a simplified perspective view of implant 10 which is removed from a delivery device such as a catheter for the purposes of clarity. Implant 10 is, in an expanded state, a coil for use in occlusion of vessels in particular vascular anomalies such as an aneurysm. Implant 10 is generally defined as a flexible element that is formed from a primary winding composed of at least two wires 12, 13 being helically wound to form a helical winding 11. Helical winding 11 is composed of at least a first wire 12 and a second wire 13 which are arranged and disposed side-by-side forming independent helical coils as a multiple helix winding. As will be described in more detail later, first and second wires 12, 13 may be wound side-by-side either simultaneously or sequentially on a common longitudinal axis such as for example a mandrel (not shown). In particular, the at least two wires 12, 13 are releasably attached along at least a portion of the flexible element. That is, and as will be described later, such a releasably attachment provides for partial dissociation of the at least two independent coils. Moreover, and will be described in more detail later, the at least two independent helical coils are disposed generally co-axially prior to release within a vascular site and are expandable to a non co-axial configuration when released within a vascular site. In such instance, the expanded non co-axial configuration expands to fill a greater volume within the aneurysm or vessel.

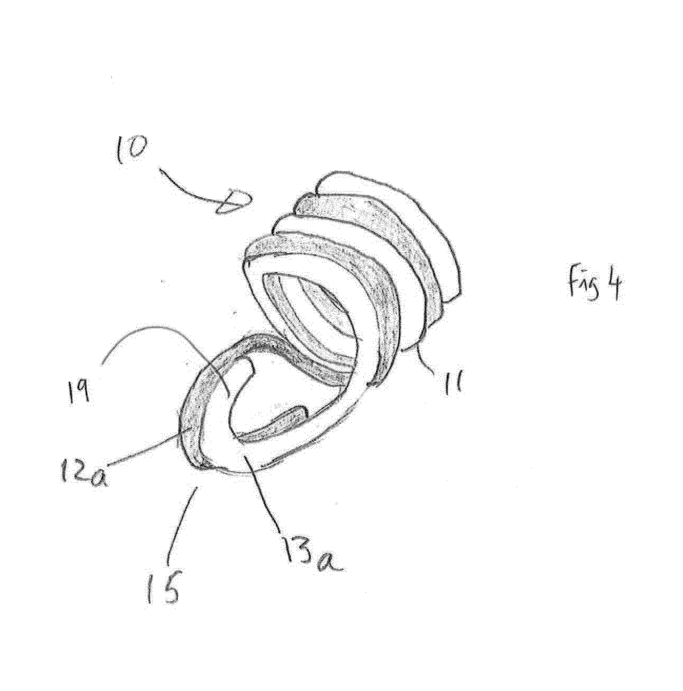

Each of the wires 12, 13 has a proximal end 12a, 13a and a distal end 12b, 13b that terminate at a proximal end 15 and distal end 16 respectively of implant 10. The proximal wire ends 12a and 13a may be terminated in a variety of ways in order to accommodate various delivery pusher means. As shown in Figure 4, the proximal ends 12a and 13a of wires 12, 13 may be simply bent at right angles at positions diametrically opposing each other on the coil to form an attachment means 19, thus constraining each independent coil generally co-axially to the form of the flexible element of implant 10. In particular, such an attachment means 19 may be provided in the form of either individual or combined hooks 19. When combined and formed in side-by-side fashion, hooks 19 form a robust enclosed loop that can be secured to a pusher (not shown). Furthermore, such a double-looped terminal configuration may allow several implants to be joined or chained together should an implant with multiple detachment zones be desirable. Thus such a configuration allows for a greater expansion to fill a greater volume within the aneurysm or vessel.

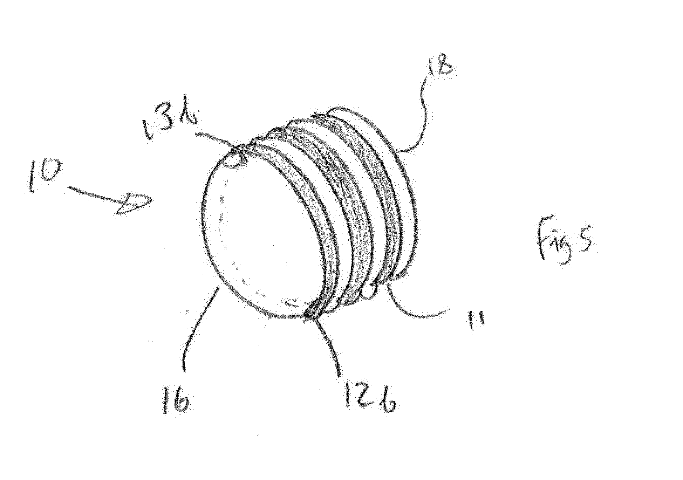

Although not clear in the present figure, distal ends 12b and 13b of wires 12, 13 can simply be stubbed and embedded in solder or adhesive to from a non-traumatic ball-shaped distal end 16 as shown in detail in Figure 5 thus minimizing the risk of inadvertent damage to surrounding vascular walls during and after deployment within a vessel.

In one aspect of the present invention, the first wire 12 may be of a different property to the second wire 13 and as can be seen for example in the current figure, such different properties may relate to different diameters of the wires. That is, such different diameter properties provide an irregular outer profile of implant 10. In another aspect however and as will be described later, first and second wires 12, 13 may differ materially from each other. For example, one of the wires may be made from a radiopaque material and another of the wires may be of a non-opaque material.

Figure 2 shows an end-on elevation view of the primary winding of Figure 1 according to an embodiment of the present invention. In particular, the smaller outer diameter coil of wire 12 is shown with its cut proximal end 12a extending 180 degrees further than the cut proximal end 13a of the larger coil formed from wire 13. In particular, such a configuration allows proximal ends 12a, 13a of wires 12, 13 to be bent at right angles at positions diametrically opposing each other on the coil to form an attachment means, thus constraining each independent coil generally co-axially to the form of the flexible element of implant 10 as is depicted more clearly in Figures 1 and 4. Such an attachment means may be provided in the form of either individual or combined hooks 19. When combined and formed in side-by-side fashion, the hooks form a robust enclosed loop that can be secured to a pusher (not shown).

Figure 3 shows a cut-away (along line A-A of Figure 2) longitudinal section view of an embodiment of implant 10 according to the present invention showing first wire 12 and second wire 13 disposed side-by-side forming a helical winding 11. As depicted in the current figure, implant 10 comprising helical winding 11 of first and second wires 12, 13 is composed of generally circular wires. However, it is envisaged that generally flat wires or any non-circular wires could be used to form such a helical winding 11. As is further depicted in the current figure, the diameter of first wire 12 may be less than the diameter of second wire 13. First and second wires 12, 13, and constrained at least at the proximal ends 12a, 13a, are arranged side-by-side and moreover are in direct contact with each other such that a common lumen 18 is provided. Both first wire 12 and second wire 13, as shown, have been wound simultaneously around a regular common longitudinal axis of, for example, a mandrel (not shown) such that the outer profile of the helical winding 11 is irregular. This irregularity defines a height difference D between outer profiles of each first and second wire 12, 13. This height difference, and as will be described later, provides an extreme outer profile of the helical winding 11 which defines a contact area for generally interfacing with an inner surface of a vessel or a catheter when transporting the implant to the vascular site (not shown). In another embodiment, first wire 12 and second wire 13 may be of similar or equal diameter but wound together around an irregular common longitudinal axis such as a mandrel. Again, the outer profile of the helical winding may be similarly irregular, thus defining a similar height difference D. In both examples however, the wires 12, 13 when wound are in direct contact with each other, and provide a common lumen 18. However, in another example, it is envisaged that the height difference D can be accentuated by providing a combination of much greater difference in first and second wire 12, 13 diameters and further by winding wires 12, 13 around a mandrel having an irregular but cylindrical longitudinal axis.

Arranged within common lumen 18 is a constraining means 17. Constraining means 17, which may for example be a stretch resistant fibre, prevents complete disassociation of the at least two independent coils during release of implant 10 within a vascular site. Constraining means 17 may extend through common lumen 18 from the proximal end to distal end of implant 10. Although not depicted in the current figure, constraining means 17 may be adhered to a part of proximal end 15 of implant 10. The properties of the constraining means 17 determine the fixture to proximal end 15 of implant 10. That is, if constraining means 17 is a fibre then an appropriate technique e.g. soldering, gluing, twisting or a combination thereof may be utilised to adhere constraining means 17 to proximal end 15 of implant 10.

Similar to the proximal end, constraining means 17 may be adhered to distal end 16 of implant 10 by means of a suitable technique, such as soldering, gluing, twisting or a combination thereof. However, to further allow expansion of the at least two independent helical coils, that is, to a non co-axial configuration within a vascular site, constraining means 17 may be removed from common lumen 18 after deployment of implant 10 within a vascular site. In such instance, the expanded non co-axial configuration may expand to fill a greater volume within the vascular site. Removal of such a constraining means 17 may for example be by pulling or cutting at a position near to proximal end 15, and/or distal end 16 of implant 10.

In another example (not shown), the wires 12, 13 may be formed into a non-circular helical winding 11 by means of forming them into a multiple helix on a mandrel of any cross-sectional shape including but not limited to circular, triangular, ovoid, square, hexagonal and so on such that the at least two independent coils have different cross-sectional configurations. Furthermore, in yet another example (not shown), the wires 12, 13 may be formed into helical shapes that are different from each other. By means of a first mandrel (not shown), as an example, first wire 12 may be formed as a circular helical winding while providing for gaps between the successive coils during the formation of the first helix. The first mandrel may then be removed and substituted with a second mandrel with a different cross-sectional size or even a different cross-sectional shape so long as it passes within common lumen 18 of the coil of first wire 12 created by the first mandrel. A helical coil from second wire 13 may then be formed to the shape of the second mandrel with successive coil loops formed intermittent to the existing coils of wire 12. Alternatively, the at least two independent coils may be formed on separate mandrels and then manually aligned within each other with successive coils of the two helixes intermittently inserted within each other to form a co-linear double helix of the two coils. Hence, either or all the wires of the composite helix may have different properties and/or independent cross-sectional configurations including but not limited to circular, triangular, ovoid, square, hexagonal and so on.

Figure 4 shows a perspective view of one embodiment of a proximal end of the implant 10 in which proximal end 12a, 13a of first wire 12 and second wire 13 of the helical winding 11 are provided as attachment means 19. That is, proximal ends 12a, 13a of wires 12, 13 may be simply bent at right angles at positions diametrically opposing each other on the coil to form an attachment means, thus constraining each independent coil generally co-axially to the form of the flexible element of implant 10. In particular, such an attachment means 19 may be provided in the form of either individual or combined hooks 19. When combined and formed in side-by-side fashion, hooks 19 form a robust enclosed loop that can be secured to a pusher (not shown). Furthermore, such a double-looped terminal configuration may allow several implants to be joined or chained together should an implant with multiple detachment zones be desirable. Thus such a configuration allows for a greater expansion to fill a greater volume within the aneurysm or vessel.

Figure 5 shows a perspective view of one embodiment of a distal end 16 of implant 10 in which the distal ends 12b, 13b of the first wire 12 and second wire 13 of the helical coils are encapsulated in a hemispherical solder mass to produce a non-traumatic distal tip. Distal ends 12b and 13b of wires 12, 13 can simply be stubbed and embedded in solder or adhesive to from a non-traumatic ball-shaped distal end 16 thus minimizing the risk of inadvertent damage to surrounding vascular walls during and after deployment within a vessel. The side-by-side arrangement of the independent coils and generally co-axially arranged to form the flexible element of implant 10 thus provide a common lumen 18. Additionally, constraining means 17 which may extend from proximal end 15 of implant 10 may be adhered to distal end 16 by means of a suitable technique, such as soldering, gluing, twisting or a combination thereof. However, it is preferable constraining means 17 is adhered to either distal end 12b and/or 13b of first wire 12 and wire 13 to allow the partial disassociation of implant 10, and thus providing a greater filling volume within the aneurysm.

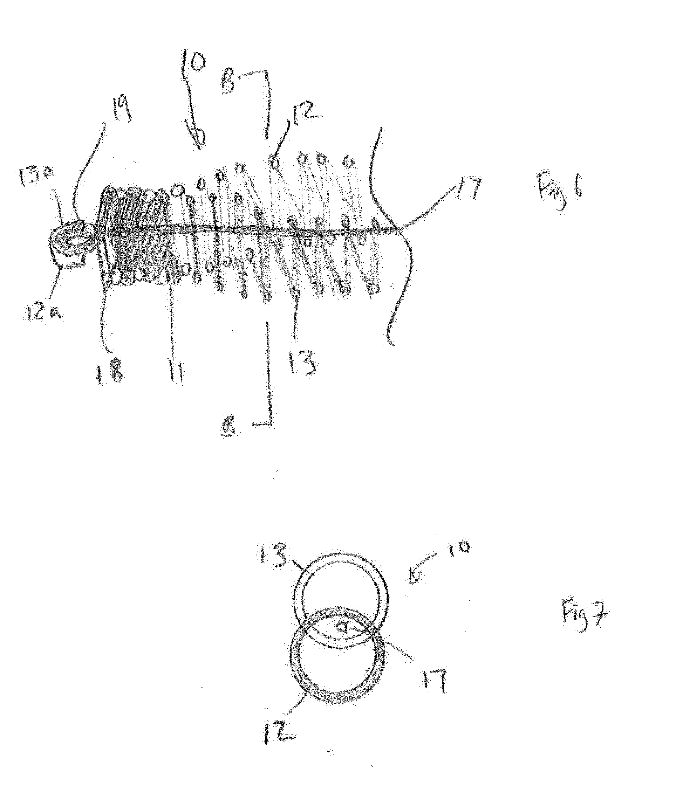

Figure 6 shows a cross-section of the implant after implantation in which the two coils have dissociated their co-linear relationship and are lengthwise constrained from further dissociation by a constraining means 17 arranged within common lumen 18. As is clearly shown in the current figure, only two independent coils are utilised to form implant 10. However, it is envisaged that any number of independent coils may be provided such that an aneurysm is effectively repaired. In the present configuration, first wire 12 and second wire 13 forming the two independent coils are disassociated and thus restrained by constraining means 17 extending from proximal end 15 of implant 10 through common lumen 18 to a distal end 16. As previously described, constraining means 17 may adhered at proximal end 15 of implant 10 by means of adhering to proximal ends 12a, 12b of first and/or second wire and further optionally, but not shown in the current figure, of adhering to distal end 16 of implant 10.

Figure 6 further shows the proximal ends 12a and 13a of wires 12, 13 which are bent at right angles at positions diametrically opposing each other on the coil to form an attachment means 19, thus constraining each independent coil generally co-axially to the form of the flexible element of implant 10. In particular, such an attachment means 19 may be provided in the form of either individual or combined hooks 19. When combined and formed in side-by-side fashion, hooks 19 form a robust enclosed loop that can be secured to a pusher (not shown). Furthermore, such a double-looped terminal configuration may allow several implants to be joined or chained together should an implant with multiple detachment zones be desirable, as previously discussed.

The implant 10 formed from at least two independent coils may take a generally co-axial configuration when disposed within a delivery catheter as shown for example in the previously described Figures 1-3. However upon deployment into a vascular site of treatment, the at least two independent coils may dissociate from each other to assume a non co-axial configuration to fill a volume greater than when in the co-linear configuration. To allow for the coil to be retractable back into the delivery catheter (such as when repositioning is desired), at least the proximal ends of each coil 12a and 13a are constrained co-axially to a primary winding 11 (not shown). When separate coils are prevented from full dissociation such as with a constraining means 17, the primary winding can thus be deployed and retracted.

Figure 7 shows an end-on elevation view along line B-B of Figure 6 in which the dissociated co-linear relationship of the two coils are further shown with a constraining means 17 arranged within a common lumen 18. In the current figure, constraining means 17 is not removed from implant 10 thus preventing complete disassociation of the at least two independent coils. Furthermore, the outer diameter of constraining means 17 is depicted to be much less than the inner diameter of first and second coils thus allowing implant 10 to expand to a non co-axial configuration when deployed within a vascular site of a patient.

As previously discussed, the first wire 12 may be of a different material to second wire 13 and in particular the first wire 12 may be composed of implantable grade Pt-W alloy. Similarly, second wire 13 may be of implantable grade Cobalt Chromium alloy. In particular, second wire 13 has a diameter that is greater than the diameter of the first wire 12 such that use of its very high specific strength properties is made, that is, to provide a highly structured, but deformable implant 10. However, it is envisaged that optionally third, and fourth wires (not shown) may additionally be added to compose the helical winding 11 such that the physical properties of the implant 10 can be varied or the expanded volume of the implanted coil may be further maximized. For example, addition of third and fourth wires may result in different stretch resistance or compact resistance of implant 10.

Of importance to users, i.e. physicians, especially during deployment of implant 10 within a vessel such as an artery or vein, is that the risk of inadvertent damage to surrounding vascular areas is minimized. To that end, and as shown in the current figure, helical winding 11 may be composed of different materials displaying different properties. That is, first wire 12 and second wire 13, wound around a regular common longitudinal axis, are of a different diameter such that the outer profile of the helical winding 11 is irregular. Accordingly, the friction generated between the outer surface of the implant 10 and an inner surface of a vessel during deployment is minimized since only one wire 13, that is the wire with the greater diameter, provides a contact area and thus a general interface with the internal surface of the vessel. Correspondingly however, and as previously discussed, it is envisaged that first wire 12 and second wire 13 are of similar diameters but sequentially wound around successive mandrels of different sizes or shapes. In this regard, the external contact area of the implant 10 and thus the general interface to an inner surface of a vessel would still be minimized thus reducing the risk of inadvertent damage within a vessel.

Preferably, at least one of either first wire 12 or second wire 13 is made of a generally resilient material such that after winding on a common longitudinal axis, implant 10 displays continuous spring-like properties that on deployment within a site of vascular anomaly, implant 10 extends and conforms to the shape of the anatomical cavity. That is, implant 10 adapts to the destination site of deployment. Depicted clearly in the cut-away figure of Figure 3 is the outer profile of the helical winding 11. Preferably, the first wire 12 may be composed of implantable grade Pt-W alloy and the second wire 13 may be of an implantable grade Cobalt Chromium alloy. Since the diameter of wire 13 is larger than and overlies the diameter of wire 12 on both sides of the coiled multiple helix, the coil formed by wire 12 is mechanically constrained within the coil formed by wire 13 helping to keep the two coils co-linear in a unified coil.

By ensuring that first and second wires 12, 13 are disposed side-by-side, and particularly terminating and constrained in such manner at both proximal 12a, 12b and optionally at distal end 13a, 13b, first and second wires remain side-by-side when implant 10 conforms to the shape of the anatomical cavity. However, the gap between wound wires may, upon conforming to the shape of the cavity, may expand.

As discussed, first and second wires 12, 13 are generally wound around a common longitudinal axis of a cylindrical mandrel (not shown). Preferably, first and second wires 12, 13 are wound nearly perpendicularly to the longitudinal axis for simplification during manufacturing. However, it can be envisaged that both first and second wires 12, 13 can be wound around the mandrel at greater angles to the longitudinal axis of a mandrel depending on the requirements of the implant 10. For example, first and second wires 12, 13 may collectively and simultaneously be wound at an angle of for example 45 degrees in relation to the longitudinal axis of a mandrel such that manipulation during deployment, that is the pulling force of the spring-like property of the implant 10 required, is less than the pulling force required if the first wire 12 and second wire 13 are wound perpendicularly to the longitudinal axis of a mandrel. Hence with, it can be envisaged that such a configuration provides an implant 10 with a higher degree of stretch resistance while providing for a high degree of flexibility.

Furthermore, and as previously discussed, at least two wires are utilised forming at least two independent coils of different properties. Since metals do not generally make for suitable drug delivery platforms, the first wire 12 may be of traditional Pt-W alloy giving the embolic device its physical and radiopacity characteristics, while the second wire may be of a polymeric material specifically chosen to enhance healing or to deliver a drug either embedded in or upon it.

## Claims

1. An implant for occlusion of vascular anomalies comprising a flexible element that is formed from a primary helical winding, wherein at least a portion of the primary helical winding is composed of at least two wires forming at least two independent helical coils as a multiple helix winding.

2. The implant of claim 1 wherein the at least two wires are releasably attached along at least a portion of the flexible element.

3. The implant of claim 2 wherein the at least two independent helical coils are disposed generally co-axially in a delivery configuration and are expandable to a non co-axial configuration when deployed.

4. An implant of any of claims 1-3 wherein each independent helical coil has distal and proximal ends disposed along the length of the flexible element, wherein at least the proximal ends of each independent coil are constrained generally co-axially to form the flexible element.

5. The implant of claim 1 wherein the at least two independent coils are constrained to have a common lumen.

6. The implant of claim 5 wherein a constraining means, in particular a stretch resistant fibre, is provided within at least a portion of the common lumen to prevent complete disassociation of the at least two independent coils.

7. The implant of claim 6 wherein the constraining means is removable to promote disassociation of the at least two independent coils.

8. The implant of any of claims 1-7 wherein the at least two wires have different properties.

9. The implant of claim 8 wherein the at least two wires are made of
different materials.

10. The implant of claim 9 wherein at least one of the wires is made from a
radiopaque material and at least another one of the other wires is made from a non-radiopaque material.

11. The implant of any of claims 8-10 wherein the different properties of
the wires include the diameter of the wires.

12. An implant of any of claims 1-11 wherein the at least two independent
coils have different cross-sectional configurations.

13. A method for producing an implant for occlusion of vascular anomalies
comprising forming a flexible element wherein at least a longitudinal portion of said element is composed of at least two independent helical coils formed into a multiple helix winding.

14. The method of claim 13 wherein the wires are simultaneously or
sequentially wound into a multiple helix onto a mandrel.

15. The method of claim 14 wherein one wire is first wound into a helix on
a first mandrel and then transferred onto a second mandrel and then at least one other wire is sequentially wound onto the second mandrel with the coil wires disposed intermittent to the previously formed wires to form a multiple helix.
